# EUROPEAN PATENT SPECIFICATION

(11) **EP 3 900 686 B1**
(45) Date of publication and mention of the grant of the patent: **06.11.2024**
(21) Application number: 20171374.0
(22) Date of filing: 24.04.2020
(51) Int. Cl.: A61F 13/475, A61F 13/49, A61F 13/511

(54) **ABSORBENT ARTICLES AND METHODS OF MAKING**
ABSORBIERENDE ARTIKEL UND VERFAHREN ZUR HERSTELLUNG
ARTICLES ABSORBANTS ET LEURS PROCÉDÉS DE FABRICATION

(43) Date of publication of application: 27.10.2021
(73) Proprietor: Ontex BV, 9255 Buggenhout (BE); Ontex Group NV, 9320 Erembodegem (BE)
(72) Inventor: Cobbaert, Dries, 1770 Liedekerke (BE); Lambertz, Christina, 56566 Neuwied (DE); Garaj, Norbert, 56727 Sankt Johann (DE); Ingenfeld, Björn, 53115 Bonn (DE); Roets, Karen, 72760 San Pedro Cholula, Puebla (MX); Van Sande, Nathalie, 9688 Maarkedal (BE); Heege, Thomas, 56761 Düngenheim (DE)
(74) Representative: Larangé, Françoise

(56) References cited:
- EP-A1- 3 403 627
- EP-A1- 3 403 632
- EP-A1- 3 620 144
- WO-A1-2018/167146
- WO-A1-2019/158226

## Description

### TECHNICAL FIELD

The present disclosure pertains to the technical field of absorbent hygiene products and relates to an absorbent core that can be used within an article for absorbing body fluids and exudates, such as urine and fecal material, or blood, menses, and vaginal fluids. In particular, the present disclosure relates to absorbent garments (or articles), such as disposable diapers or diaper pants, disposable incontinence diapers or pants, which are configured to collect and contain fecal material and avoid leakage, or sanitary napkins or panty liners, which are configured to collect and contain blood, menses, urine, vaginal fluids and avoid leakage. More particularly the present disclosure relates to absorbent cores having one or more channels therethrough.

### BACKGROUND

Absorbent cores have been subject to considerable improvement and innovation over time to address needs such as improved fluid absorption and distribution, as well as comfort, and a need for continued improvement exists. Such needs are ever present in today's demanding consumer environment.

In this context, more recently, absorbent cores having one or more channels substantially free of absorbent material have been developed.

EP3342386 discloses an absorbent core comprising substantially continuous zones of one or more high fluid distribution structures and discontinuous zones of fluid absorption structures surrounding the one or more high fluid distribution structures, wherein the one or more high fluid distribution structures are arranged to distribute fluid across the absorbent core at a speed that is faster than the speed of fluid distribution across the absorbent core by said discontinuous fluid absorption structures, and wherein said continuous zones extend along a path that is substantially parallel to at least a portion of the perimeter of the core, said portion of the perimeter of the core comprising at least a portion of the sides of the core and one of the ends of the core. In particular it describes one or more high fluid distribution structures consisting of two nonwoven webs bonded together.

WO2019/158226 discloses an absorbent core particularly designed to improve uniform liquid distribution and comfort, comprising at least one interconnected channel substantially free of absorbent material, formed by mechanically bonding said upper layer directly to said lower layer at a plurality of distinct bonding sites. The channel has at least portions extending along both the length and the width of said core arranged to form an open end proximal to the front portion and a closed end proximal to the back portion. It also describes a channel shape having a crossing point.

EP3620144 discloses elasticised absorbent articles with channelled cores comprising one or more longitudinally extending channels substantially free of absorbent material. Amongst various possible channel designs, a channel shape having a crossing point is disclosed.

EP3403632 discloses an absorbent core provided with a plurality of attachment zones where the top core wrap sheet is attached to the back core wrap sheet, and which upon wetting of the absorbent core create channels. In particular it describes a first and a second elongate attachment zone which are crossing the longitudinal center line. The first elongate channel extends from a first left position to a second right side, where the first left position is closer to the first transverse edge than the second right position. Similarly, the second elongate channel extends from a second right position to a first left position, where the second right position is closer to the first transverse edge than the first left position. This is said to create a convenient liquid distribution channel network allowing the liquid to be distributed rapidly throughout the absorbent core.

WO2019/152005 discloses absorbent bodies aimed at reducing the crotch bulk, including a reduced absorbent section having less absorbent material than an adjacent section of the absorbent. The reduced absorbent section includes a rear diverging section including a first arm and a second arm, and a front diverging section with a third arm and a fourth arm. However no attachment of webs is described within the reduced absorbent section so as to create channels as herein considered.

Although channels are beneficial in terms of fluid handling, there still remains a need to further improve liquid distribution, whilst ensuring comfort and avoiding leakage.

The present disclosure aims to provide a novel absorbent article utilizing a channeled core particularly designed to provide exceptionally fast absorption and good liquid distribution throughout the entire core, leading to improved absorption capacity and low rewet that provides to the consumer an enhanced perception of dryness. It further offers an improved fit to the wearer, thereby providing comfort and minimizing the risk of leakage.

### SUMMARY

In one aspect, the present disclosure relates to an absorbent article according to claim 1 or claim 2.

We have found that having a longitudinally extending central section coincident with the longitudinal center line, i.e. of a certain length rather than a crossing point, greatly improves the absorption time, providing shorter absorption times under pressure for example. We also believe that this longitudinally extending central section is advantageous for providing some tolerance in the placement of the absorbent article, as the length of the section may cover various positioning of the miction point.

The lateral sections greatly participate in the distribution of the fluid in the fourth quadrants of the core, for a distribution through the entire core. Their extension in the longitudinal direction helps guiding the fluid to the front and back of the absorbent core.

We have found that the diagonal sections help in providing a good cup shape and 3D bending of the absorbent article once worn. This provides comfort to the user and may also reduce the risk of leakage through the longitudinal edges in the crotch region. We have indeed noted that with channels of the prior art which mainly extend longitudinally as a pair of parallel channels, the absorbent article may tend to wind on itself in the crotch region, i.e. the longitudinal edges wrapping over the longitudinal center line between the legs of the user, thereby increasing the risk of leakage on the sides of the absorbent article between the user's legs. The diagonal sections may help in preventing such wrapping.

Finally, we have found that having a transversally extending closing section may help the distribution of liquid beyond simply the end points of the lateral sections closest to the transverse edges. The closing section may allow distribution of fluid beyond the closing section itself, closer to the transverse edge of the core.

A continuous channel made of at least these essential channel sections provides fast absorption, good liquid distribution throughout the entire core, improved fit and comfort and minimizes the risk of leakage. It also contributes to limit over-saturation of the core in the portion of fluid discharge. Without wishing to be bound by theory it is believed that the fact that the fluid is distributed across the core and immediately away from the fluid discharge position, a perception of dryness and skin comfort is provided to the wearer, as well as an impression of longer lasting dryness by the user.

Other objects and advantages of this invention will become apparent hereinafter.

### BRIEF DESCRIPTION OF THE FIGURES

Fig. 1 to 4 show diagrammatic top views of absorbent cores according to embodiments herein.
Fig. 5 to 9 show top views of absorbent cores according to embodiments herein.
Fig. 10 shows a top view of a wetted absorbent core not in accordance with the present invention.
Fig. 11 shows a top view of a wetted absorbent core in accordance with the present invention.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

An absorbent article, such as a diaper, comprises a front waistband region, a back waistband region, an intermediate crotch region which interconnects the front and rear waistband regions. When used herein, reference to a "front" portion refers to that part of the absorbent article which is generally located on the front of a subject, such as an infant or adult, when in use. Reference to the "rear" or "back" "portion" or "section" refers to the portion of the absorbent article generally located at the rear of the subject, such as an infant or adult, when in use, and reference to the "crotch" portion refers to that portion which is generally located between the legs of subject, such as an infant or adult, when in use. The crotch region is an area where repeated fluid surge typically occurs, within the absorbent article assembly.

"Front", "rear or back", and "crotch" portions of the absorbent core as used herein typically refer to portions of the absorbent core that are proximal to respective portions of the absorbent article. For example, the "front" portion of the core is that which is most proximal to the front of the subject when worn, the "rear or back" portion of the core is that which is most proximal to the rear or back of the subject when worn, and the "crotch" portion of the core is the middle portion of the absorbent core between the "front" and "rear or back" portions.

Preferably, a diaper comprises a liquid permeable "top sheet", a liquid impermeable "back sheet", and an "absorbent medium" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means as are known in the art.

"Acquisition and distribution layer", "ADL", "Acquisition and distribution system" or "surge management portion" refers to a sub-layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "bulk density" as used herein refers to the weight of a material per unit of volume. Bulk density is generally expressed in units of weight/volume (e.g., grams per cubic centimeter). The bulk density of flat, generally planar materials such as, for example, fibrous nonwoven webs, may be derived from measurements of thickness and basis weight of a sample. The basis weight of the sample is determined essentially in accordance with ASTM D-3776-9 with the following changes: 1) sample size is cut to 10.16 cm X 10.16 cm (4 inches X 4 inches) square and 2); a total of 9 samples are weighed.

The term "adhesive" as used herein is intended to refer to any suitable hot melt, water or solvent borne adhesive that can be applied to a surface of a product component of the present disclosure. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present disclosure can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present disclosure. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

As used herein, an "air-through-bonded" nonwoven, is a nonwoven structure primarily formed by a process that comprises the application of heated air to the surface of the nonwoven fabric. During the through air bonding process, heated air flows through holes in a plenum above the nonwoven material. Unlike hot ovens, which push air through the material, the through air process uses negative pressure of suction to pull the air through an open conveyor apron holding nonwoven as it is drawn through the oven. Pulling air through the material allows the rapid and even transmission of heat to minimize distortion of the nonwoven material. The binding agents used in the through air bonding process include crystalline binder fibers and powders, which melt to form molten droplets throughout the cross-section of the nonwoven. As the material is cooled, bonding occurs at these droplet points.

As used therein, the term "associated" encompasses configurations in which top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by attachment means such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described attachment means may also be employed to interconnect and assemble together the various other component parts of the article described herein.

The term "back sheet" or "backsheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The term "blend" means a mixture of two or more polymers while the term "alloy" means a sub-class of blends wherein the components are immiscible but have been compatibilized.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to films having a water vapor transmission rate (WVTR) of at least 300 grams/m² - 24 hours.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles. The meltblown fibers containing wood fibers are collected on a forming surface, such as provided by a foraminous belt. The forming surface may include a gas-pervious material, such as spunbonded fabric material, that has been placed onto the forming surface.

"Compression" refers to the process or result of pressing by applying force on an object, thereby increasing the density of the object.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "density" or "concentration" when referring to the absorbent material, in particular the SAP, of a layer, refers to the amount of the absorbent material divided by the surface area of the layer over which the absorbent material is spread out.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent), as represented by the following: "Extension" means the change in length of a material due to stretching (expressed in units of length).

As used herein the term "extensible" means elongatable in at least one direction, but not necessarily recoverable.

The term "fabrics" is used to refer to all of the woven, knitted and nonwoven fibrous webs.

As used herein, the term "garment" means any type of apparel which may be worn. This includes diapers, training pants, incontinence products, surgical gowns, industrial workwear and coveralls, undergarments, pants, shirts, jackets and the like.

Many of the known superabsorbent polymer particles exhibit gel blocking. "Gel blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel blocking can be a particularly acute problem if the superabsorbent polymer particles do not have adequate gel strength and deform or spread under stress once the particles swell with absorbed fluid.

The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

The crotch portion of the absorbent article preferably comprises opposite longitudinal side portions which comprise a pair of elasticized, longitudinally-extending "leg cuffs". The leg cuffs are generally adapted to fit about the legs of a wearer when in use and serve as a mechanical barrier to the lateral flow of body exudates. Leg cuffs are elasticized by leg elastics. The diaper further can comprise a front waist elastic and a rear waist elastic. Materials suitable for use in forming leg elastics are known to those skilled in the art. Exemplary of such materials are strands or ribbons of a polymeric, elastomeric material which are adhered to the diaper at the leg cuff while in a stretched position, or which are attached to the diaper while the diaper is pleated, such that elastic constrictive forces are imparted to the leg cuff. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

"Liquid" means a nongaseous substance and/or material that flows and can assume the interior shape of a container into which it is poured or placed.

"Longitudinal" is a direction running parallel to the maximum linear dimension of the article.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" refers to any material which does not fall within the definition of "elastic" above

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

"Pant body" or "pant" refers to a garment that has a waist opening and a pair of leg openings, similar to shorts, swim wear, or the like. The described garment may or may not have a manually tearable side seam.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

"Pulp fluff' or "fluff pulp" refers to a material made up of cellulose fibers. The fibers can be either natural or synthetic, or a combination thereof. The material is typically lightweight and has absorbent properties.

The "retention portion" or "liquid absorption layer" is part of the absorbent medium. This portion may comprise a matrix of hydrophilic fibers, such as a web of cellulosic fluff, mixed with particles of high absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness of the absorbent structure, with lower concentrations toward the bodyside of the absorbent structure and relatively higher concentrations toward the outerside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

As used herein the term "sheet" or "sheet material" refers to woven materials, nonwoven webs, polymeric films, polymeric scrim-like materials, and polymeric foam sheeting.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed inline by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. Stretch may be unidirectional, bi-directional, or multi-directional. The specific stretch properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

As used herein "channels" are fluid distribution means within the absorbent core adapted to favour exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

By "substantially", it is meant at least the majority of the structure referred to. For example, with reference to a channel following "a substantially continuous path from any point of said channel to any other point of the same channel", this means that the majority of the channel is interconnected and generally wherein a direct and continuous path can be traced by starting from one point of the channel towards another point of the channel.

By "directly over", it is meant that the feature referred to is placed over the structure referred to such that the two are in direct contact with each other at least throughout a substantial portion of said structure.

By "indirectly over", it is meant that the feature referred to is placed over the structure referred to but in such a way that the two are not in direct contact with each other at least throughout a substantial portion of said structure. For example, a nonwoven web applied indirectly over a three-dimensional absorbent material comprises a further layer of material between said nonwoven web and said three-dimensional absorbent material.

Use of the term "substrate" includes, but is not limited to, woven or nonwoven webs, porous films, ink permeable films, paper, composite structures, or the like.

The terms "Superabsorbent" or "high absorbency" refer to materials that are capable of absorbing at least 10 times their own weight in liquid. Superabsorbent materials suitable for use in the present disclosure are known to those skilled in the art, and may be in any operative form, such as particulate form, fibers and mixtures thereof. Generally stated, the "superabsorbent material" can be a water-swellable, organic or inorganic, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal and ammonium salts of polyacrylic acid and polymethacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, maleic anhydride copolymers with vinyl ethers and alpha-olefins, poly(vinyl pyrrolidone), poly(vinylmorpholinone), poly(vinyl alcohol), and the like, and mixtures and copolymers thereof. Other suitable hydrogel-forming polymers include hydrolyzed acrylonitrile grafted starch, acrylic acid grafted starch, and isobutylene maleic anhydride copolymers and mixtures thereof, methyl cellulose, carboxymethyl cellulose, hydroxypropyl cellulose, and the natural gums, such as alginates, xanthan gum, locust bean gum and the like. The hydrogel-forming polymers may be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, irradiation, crystalline domains, covalent bonds, ionic complexes and associations, hydrophilic associations, such as hydrogen bonding, and hydrophobic associations or Van der Waals forces. Mixtures of natural and wholly or partially synthetic absorbent polymers can also be used. Synthetic hydrogel-forming materials typically are xerogels which form hydrogels when wetted. The term "hydrogel", however, has commonly been used to also refer to both the wetted and unwetted forms of the material. The superabsorbent material may be in any of a wide variety of geometric forms. As a general rule, it is preferred that the superabsorbent material be in the form of discrete particles. However, the superabsorbent material may also be in the form of fibres, flakes, rods, spheres, needles, spiral or semi-spiral, cubic, rod-like, polyhedral, or the like. Conglomerates of particles of superabsorbent material may also be used. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "top sheet" or "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

"Training pants" are available for use by children in the potty-training stage, and are popular with mothers and caretakers. A training pant typically comprises a top sheet, a back sheet, an absorbent medium between the top sheet and the back sheet, and side seams that bond portions of the side edges of the pant together to form waist and leg openings.

As used herein, the term "transverse" or "lateral" refers to a line, axis, or direction which lies within the plane of the absorbent article and is generally perpendicular to the longitudinal direction.

"Ultrasonic welding or bonding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

"Dry-state" refers to the condition in which an absorbent article has not yet been saturated with exudates and/or liquid.

"Wet-state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

By the term "concentration" of (e.g. super absorbent polymer) as used herein, is meant the amount of the referred material divided by the surface area of the layer referred to (typically said area being in the plane of the length and width of the core) over or within which the referred material is contained. This may be determined by standard weighing and dimensional measuring methods known in the art and can be expressed in g/mm².

By the term "substantially U-shaped" as used herein, is meant any shape that visually approximates the shape of a "U", such as a "V-shape", a semi-circle, and the like.

By the term "distinct cellulosic fibers" as used herein, is meant cellulosic fibers that are not part of a substrate (e.g. a nonwoven layer) and are rather distinct thereof and/or physically separated therefrom, and typically are in the form of cellulosic fibers that are enclosed though kept separate from said substrate. For sake of clarity, cellulosic fibers present within a substrate (e.g. a nonwoven layer) are not encompassed within its meaning.

By the term "substantially matches the shape of the channel(s)" as used herein, is meant that the feature referred to has an overlapping shape that is visually the same as the channel(s).

By the term "superabsorbent polymer fibers" as used herein, is meant fibers made from superabsorbent polymers (as opposed to particles made therefrom). Examples of suitable fibers for use herein are selected from those of Example 1, Example 2, Example 3, and/or Example 4 (page 5, lines 1-46) of EP3190216A1, incorporated herein by reference. Said fibers typically being used to form nonwoven webs or substrates according to the same referenced application.

By the terms "longitudinally-, transversally-, diagonally- -extending" as used herein, is meant a general orientation substantially parallel respectively to the longitudinal axis, to the transversal axis, and to any axis between them. This covers deviations from the axis used as reference of between 0° and 20°. This may cover straight lines but also curved lines. In the latter case, it is the main general orientation of the curve which is meant to be described.

By the term "center line" as used herein, is meant an axis dividing the element to which it refers, in two portions of equal length on either side of this axis.

Embodiments of the articles and processes according to the disclosure will now be described. It is understood that technical features described in one or more embodiments maybe combined with one or more other embodiments without departing from the intention of the disclosure and without generalization therefrom.

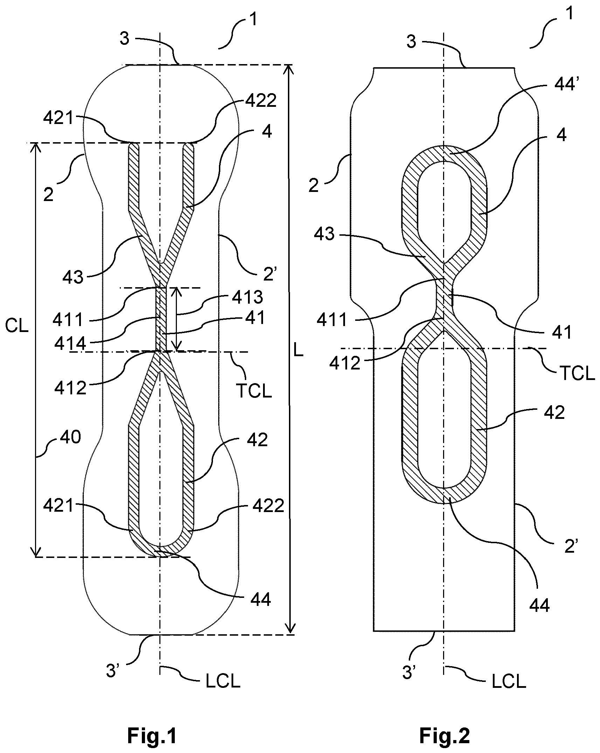

Absorbent articles according to the invention comprise a liquid permeable topsheet, a liquid impermeable backsheet and an absorbent core positioned in between said topsheet and said backsheet. The absorbent core (1), as for example illustrated in Figure 1, has a first and second longitudinal edge (2,2') and a front and back transverse edge (3,3'). It has a longitudinal center line (LCL) dividing the absorbent core (1) in a first longitudinal portion and a second longitudinal portion on either side of the longitudinal center line (LCL). It also has a transverse center line (TCL) dividing the absorbent core (1) in a front portion and a back portion on either side of the transverse center line (TCL). The longitudinal center line (LCL) and the transverse center line (TCL) together create four quadrants within the absorbent core (1). The absorbent core (1) comprises absorbent material selected from the group consisting of cellulose fibers, superabsorbent polymers and combinations thereof, and the absorbent material is contained within at least one core wrap substrate enclosing said absorbent material. The top layer of said core wrap is adhered to a bottom layer of said core wrap to form one or more channels (4) substantially free of said absorbent material. At least one channel (4) follows a substantially continuous path from any point of said channel to any other point of the same channel. Said channel (4) comprises at least:
- one longitudinally extending central section (41) coincident with the longitudinal center line (LCL),
- one longitudinally extending lateral section (42) in each of the four quadrants,
- four diagonally extending diagonal sections (43), diverging from the central section end points (411,412) towards the lateral sections in each quadrant and connecting said central (41) and lateral sections (42), and
- one transversally extending closing section (44,44') in one of the front or back portions, joining each of the two lateral sections end points (421,422) closest to the absorbent core respective front or back transverse edge (3,3').

In an embodiment, as for example illustrated in Figure 2, the channel (4) comprises a second transversally extending closing section (44,44'), in the other of the front or back portions, joining each of the two lateral sections end points (421,422) closest to the absorbent core respective front or back transverse edge (3,3'). This may allow distribution of fluid beyond the second closing section itself, closer to the transverse edge of the core.

In an embodiment, as illustrated in Figure 1, the channel (4) consists of one central section (41), four lateral sections (42), four diagonal sections (43) and one closing section (44). In that case, preferably, the closing section is in the back portion of the absorbent core, since this portion is generally the most difficult to distribute liquid as being the farthest from the miction point.

In another embodiment, as illustrated in Figure 2, the channel (4) consists of one central section (41), four lateral sections (42), four diagonal sections (43) and two closing sections (44,44'), in order to benefit from the advantages of the closing sections hereinabove described, in both the front and the back of the absorbent core.

In these embodiments where the channel consists of one central section, four lateral sections, four diagonal sections and one or two closing section(s), the central section is preferably longitudinally centered on a point (414) which is at a distance from the front transverse edge (3) along the longitudinal center line (LCL) of between 30% and 55%, more preferably between 35% and 53%, even more preferably between 40% and 50%, of the absorbent core length (L). This advantageously allows the miction point positioning to correspond to the central section, so that liquid distribution towards front and back may quickly start.

In these same embodiments, the central section (41) has a length (413) of between 4 and 25%, preferably between 5 and 20%, more preferably between 6 and 15 %, most preferably between 7 and 12%, of the absorbent core length (L). We have indeed found that if the central section is too short, the absorption time may increase and rewet, to some extent, also may increase. When the central section is too long, it has been found that both absorption time and rewet may greatly increase. Advantageously the length of the channel (CL) taken along the longitudinal center line (LCL) is between 50 and 90%, preferably between 55 and 80%, more preferably between 60 and 75%, of the absorbent core length (L).

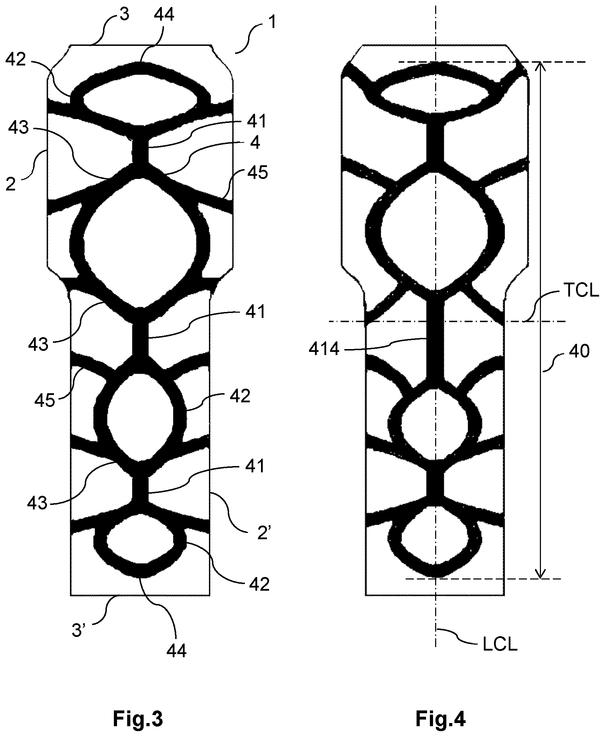

In alternative embodiments, as illustrated in Figures 3 or 4, the channel (4) comprises at least 2 central sections (41), 6 lateral sections (42), 8 diagonal sections (43) and two closing sections (44). Such pattern may provide a powerful draining system and/or comfort to the wearer. We have indeed found that all the channel sections may work together to offer powerful liquid distribution and may act as folding lines for the core, and the article including such core, to fit to the wearer body.

In these alternative embodiments where the channel comprises at least 2 central sections, the central section (41) closest to or crossing the transverse center line (TCL), preferably the central section closest to or including the miction point, is preferably longitudinally centered on a point (414) which is at a distance from the front transverse edge along the longitudinal center line (LCL) of between 40% and 65%, more preferably between 45% and 60%, of the absorbent core length (L). This advantageously allows the miction point positioning to correspond to the central section, so that liquid distribution towards front and back may quickly start.

In these alternative embodiments, the central section (41) closest to or crossing the transverse center line (TCL), preferably the central section closest to or including the miction point, has a length (413) of between 5 and 25%, more preferably between 10% and 20%, of the absorbent core length (L). We have indeed found that if the central section is too short, the absorption time may increase and rewet, to some extent, also may increase. When the central section is too long, it has been found that both absorption time and rewet may greatly increase.

In these alternative embodiments, the channel (4) may advantageously further comprise at least two diverging sections (45) connecting lateral sections (42) and/or diagonal sections (43) to the longitudinal edges (2,2') of the absorbent core (1). These diverging sections may further help the liquid distribution through the entire core and/or improve the comfort and fit. Preferably, however, the absorbent core is free of any diverging section connecting the channel to the front or back transverse edges to reduce the risk of leakage in these zones.

In these alternative embodiments, the length of the channel (CL) taken along the longitudinal center line (LCL) is preferably between 70 and 95%, more preferably between 80 and 95%, even more preferably between 83 and 93% of the absorbent core length (L).

In these alternative embodiments, the ratio of the channel surface area, i.e. the surface area substantially free of absorbent material (in black in figures 3 or 4), to the surface area of absorbent material (in white in figures 3 or 4) is preferably between 20 and 50%, more preferably between 30 and 40%. It has indeed been found that when this ratio is too low there might be not enough or not efficient enough fluid transport, and if the ratio is too high there might be a greater likelihood of leakage as liquid will flow faster to the edges of the core compared to the speed of acquisition into the absorbent material.

In accordance with the invention, the channel sections may be straight lines or may be curved lines. Preferably, the closing section is substantially curvilinear in shape, for example in the form of a U-bend with its convex side facing the absorbent core transverse edge at closest proximity, or is substantially linear in shape, for example forming a straight or triangular shape between the lateral sections end points.

According to the invention, preferably, the absorbent core is free of any channel or channel section extending up to the front and back transverse edges of the absorbent core. This has been found advantageous in that it creates transversally extending zones of absorbent material acting as barriers to absorb liquid before it reaches the front and back end of the core, thereby avoiding leakage in those generally risky areas of the core. A similar advantage may be reached, alternatively or additionally, by ensuring that the concentration of superabsorbent polymers within the core is higher closer to the front and back transverse edges compared to the central region closer to the transverse center line, for example by ensuring an increasing gradient of superabsorbent polymers concentration from the central portion of the core to the front and back portions. Alternatively or additionally, it might be desirable to have a concentration of superabsorbent polymers within the core higher around the miction point. This may offer a reservoir for absorption in order to minimize risk of leakage.

Advantageously, the lateral sections (42) of the first longitudinal portion may be aligned on a first longitudinal axis parallel to the longitudinal center line (LCL), and the lateral sections (42) of the second longitudinal portion may be aligned on a second longitudinal axis parallel to the longitudinal center line (LCL). This may render the manufacturing process easier, ensuring the adhesion of the core wrap top and bottom layers along lines parallel to the manufacturing direction. Preferably the channel is symmetric about the longitudinal center line.

According to the invention, the one or more channels are formed by the adhesion of the top layer of the core wrap with the bottom layer of said core wrap, in zones substantially free of absorbent material. Such adhesion may advantageously provide core stability and for example reduce the risk of sagging. This adhesion may be provided by any means known in the art to bond core wrap layers together, for example with adhesive or mechanical bonding.

In an embodiment, the top and bottom core wrap layers are adhered by one or more adhesives. The adhesive may be either uniformly applied within the channels or it may be applied in zones of the channels such to form zones, preferably alternating zones, of different bonding strength between the core wrap layers. Ways to achieve stronger bonding strength in some zones may include using higher amounts of adhesive in said zones, applying greater mechanical pressure on said zones, or utilizing a different adhesive type.

In an embodiment, the top and bottom core wrap layers are adhered along the channels, at a plurality of discrete joining areas, preferably wherein said discrete joining areas are free of adhesive and typically comprise mechanical bonds. Advantageously, this allows for a permanent bonding of the top and bottom layers of the core wrap that limits the presence of additional hydrophobic substances such as adhesives therebetween whilst maximizing the unbonded spaces therebetween to enhance the fluid flow through the channel.

In an embodiment the bonding strength in some zones of the channels is less than in others, and the top core wrap layer and bottom core wrap layer may separate in said zones. This arrangement may allow the bonding in some zones to fail upon for example swelling of the SAP such to allow more volume to be available for expansion thereof (and prevent early saturation or non-optimal absorption), with typically the zones resisting such expansion providing integrity of the channels even in wet state.

The core wrap layers are preferably nonwoven webs. Advantageously, at least one of the top and bottom core wrap layers is an elastic nonwoven (e.g. containing an elastic material such as Vistamaxx resin from ExxonMobil, or other suitable polymers capable of imparting elasticity to a nonwoven web). An advantage of this embodiment is that the nonwoven web better and more easily wraps around the 3D insert during the manufacturing process.

According to the invention, the absorbent core comprises absorbent material selected from the group consisting of cellulose fibers, superabsorbent polymers and combinations thereof. The absorbent material may in particular comprise cellulosic fluff and/or fibrous web typically comprising airlaid fibers of the cellulosic kind. The superabsorbent polymers may typically be in the form of a plurality of discrete particles that may be distributed within the absorbent material or directly agglomerated in one or more pockets between at least two nonwoven webs. The absorbent core may include cellulose fibers and superabsorbent polymers in a proportion of 5-55 Wt% cellulose and 45-95 Wt% SAP.

In an embodiment, in addition to the channel having the sections as described above, the core further comprises one or more disconnected channels, an advantage being effective added local uniform fluid distribution.

In an embodiment, the width of the channels is constant throughout a continuous path of a channel. Alternatively, it may vary along a channel. The width of the channels may be between 3 and 20 mm, preferably between 5 and 15 mm, more preferably between 5 and 12 mm.

The cores herein may have various shapes. Preferably the width of the core in the region of the transverse center line is less than in at least one of the front or back portions. This region may be positioned in a crotch portion of the absorbent article such to provide better ergonomics and fit along the leg of a wearer. It is preferred that said cores are symmetric at least about the longitudinal axis thereof. Irrespective of the core geometry, it is understood herein that the same or similar channels as described herein may be interchangeably used.

In an embodiment, the absorbent core is a multi-layer core comprising at least a first and a second distinct core layers positioned one on top of the other. The first and/or the second core layer may comprise at least one channel according to the invention. Preferably they both comprise at least one channel according to the invention, wherein the shape and/or dimensions and/or positioning of said channel is substantially identical in both core layers.

Advantageously, a first core layer comprises a first concentration of superabsorbent polymers therein and a second core layer comprises a second concentration of superabsorbent polymers therein. Said first and second concentrations may be different. The multi-layered core arrangement with differential superabsorbent polymer concentration enables to more efficiently absorb fluids in a multi-fold way and reduce the risk of gel-blocking. The second concentration of superabsorbent polymers, in the bottom core layer, may be greater than the first concentration of superabsorbent polymers, in the top core layer. Preferably said second concentration is at least 1.5, preferably 2, times greater than the first concentration. Such brings advantages such as reduced risk of gel-blocking. The type of superabsorbent polymers may be selected to ensure that the top core layer includes SAP performing well under pressure, to provide a better rewet, and that the bottom core layer includes SAP having fast absorption characteristics. The top core layer may include cellulose fibers and superabsorbent polymers in a proportion of 20-40 Wt% cellulose and 60-80 Wt% SAP; the bottom core layer may include cellulose fibers and superabsorbent polymers in a proportion of 15-30 Wt% cellulose and 70-85 Wt% SAP.

Absorbent articles according to the present invention include disposable diapers or diaper pants; disposable incontinence diapers or diaper pants; sanitary pads, sanitary napkins and panty liners. They comprise a core sandwiched between a liquid permeable topsheet and a liquid impermeable backsheet. The backsheet may comprise a print or graphic viewable from the garment facing side of said article that substantially matches the shape and/or contour of the channel(s). This has the advantage to further accentuate the visual perception of the presence of such channel and its location in the absorbent article.

The absorbent articles may further comprise an acquisition distribution layer (ADL), also referred to herein as acquisition and distribution system, positioned between said topsheet and said core. The ADL may be positioned at a body-facing side of the absorbent core, between the topsheet and the absorbent core of the absorbent article, and more preferably in close proximity or even in good contact (most preferably in direct contact) with the body-facing side of the absorbent core. The use of an ADL in combination with the channels of the present invention may lead to an extremely good distribution of fluids from a discharge area to the entire absorbent core whilst attaining excellent perceived dryness performance.

The acquisition distribution system may be a single layer of spunbond and/or carded (e.g. carded thermobonded) nonwoven. Alternatively, the acquisition distribution system may be multi-layered and comprise at least one spunbond layer and at least one meltblown layer typically the layers being nonwoven layers. Preferably, the acquisition distribution system is a nonwoven selected from the group consisting of: SM, SMS, SMMS, and combinations thereof.

In an embodiment, the acquisition distribution layer comprises a plurality of layers, wherein at least one of said layers, preferably each of said layers, consists of spunbond, meltblown and/or carded (e.g. thermocarded) nonwoven and wherein at least the layer most distal from the body-facing side of the absorbent core consists of spunbond and/or carded nonwoven, preferably wherein both the layer most distal and the layer most proximal to the body-facing (also referred to herein as "skin-facing") side of the absorbent core consists of spunbond and/or carded nonwoven.

In an embodiment, the acquisition distribution layer for use herein comprises synthetic fibers which are comprised at a level of greater than 80%wt by weight of said acquisition distribution layer. The acquisition distribution layer may have a basis weight of from 5 to 50 g/m², 10 to 50 g/m², preferably from 15 to 40 g/m², more preferably from 18 to 35 g/m², even more preferably from 20 to 30 g/m², most preferably from 21 to 25 g/m².

In an embodiment, the acquisition distribution system is the top layer of the core wrap and the absorbent article is free of additional layers, such as an acquisition distribution layer, so that the top layer of the core wrap is in direct contact with the topsheet. Said top layer of the core wrap may comprise a spunbond and/or carded, e.g. carded thermobonded, nonwoven layer comprising synthetic fibers, wherein preferably said synthetic fibers are comprised at a level of greater than 80%wt by weight of said layer, and/or wherein said top layer of the core wrap has a basis weight of from 5 to 50 g/m². Alternatively, said top layer of the core wrap may be multi-layered and comprise at least one spunbond layer and at least one meltblown layer typically the layers being nonwoven layers. Preferably, the multi-layered top layer of the core wrap acting as acquisition distribution system is a nonwoven selected from the group consisting of: SM, SMS, SMMS, and combinations thereof. Advantageously, by choosing core wraps as described, improved performance on rewet may be achieved even whilst eliminating the presence of further acquisition distribution layers, thus further introducing cost benefits.

We have found that fluid distribution, in embodiments of the present absorbent articles which comprise an ADL, may depend on the relative size and positioning of the ADL with respect to the fluid distribution structure, and in particular the channel(s), of the absorbent core. The acquisition distribution layer may be asymmetrically positioned over the absorbent core, offset at least along the longitudinal axis, preferably positioned such that it does not overlap a portion of the channel at a position proximal to the back of the core, more preferably wherein the acquisition distribution layer is positioned such that it does not overlap with a closing section of the channel. This arrangement has the advantage of raw material cost saving by ensuring the ADL is positioned where it is needed most. Moreover by ensuring a part of the channel remains exposed (particularly the closing section of the channel) speed of liquid flow through the channel from front to back is not compromised.

In a further aspect, the present disclosure relates to a process of making an absorbent core as herein described, comprising the steps of:
i. providing a mold comprising a non-porous 3D insert therein, typically said insert having the inverse shape of the desired channel(s), wherein the mold is in fluid communication with an under-pressure source except for said insert;
ii. applying a first nonwoven web to said mold;
iii. applying an absorbent material over at least a portion of said nonwoven web;
iv. removing said absorbent material from areas of the nonwoven web corresponding to said insert, such as by the under-pressure source being arranged to provide a vacuum force forcing said absorbent material around the insert to substantially evacuate a surface of the nonwoven web in contact thereto from said absorbent material or by mechanical means such as use of a brush;
v. applying a second nonwoven web directly or indirectly over the absorbent material, or folding said first nonwoven web, such to sandwich said absorbent material between an upper and lower layers of said nonwoven web(s);
vi. optionally applying a bonding step to form a laminate comprising said first nonwoven, said second nonwoven and said absorbent material therebetween;
vii. optionally removing said laminate from the mold to form an absorbent core comprising channel(s) having the inverse shape of said insert.

Further embodiments of such process are for example described in EP3342386 A1, in paragraphs [0148] to [0160] and with reference to Figures 15A and 15B.

In a preferred embodiment, the bonding step comprises applying an adhesive on a surface of the second nonwoven web and joining said web to said first nonwoven web and/or absorbent material, preferably the adhesive being applied in continuous or discontinuous spaced apart stripes aligned with said channels such that the resulting core laminate comprises adhesive rich and adhesive poor regions, wherein the adhesive rich regions are substantially located along said channels and the adhesive poor regions are located in areas of the core other than said channels. An advantage of this embodiment is to limit the risk of adhering absorbent material within the channels and to rather directly bond the topsheet and backsheet nonwoven together at these channel locations.

In an embodiment, the mold comprises a plurality of perforations or openings across its surface typically forming channels arranged to be in fluid (preferably air) communication with the under pressure source. Preferably, the 3D insert is positioned above and/or over said mold surface comprising a plurality of said perforations or openings and said 3D insert being free of said perforations or openings and consists of a solid component that is not in fluid communication with the under pressure source.

Preferably, the 3D insert has a cross-sectional shape selected from the group consisting of square, rectangular, oval, semi-circular, and combinations thereof.

Alternatively to the use of a 3D insert, a process of making an absorbent core as herein described, in particular including a channel having a more complex shape and including numerous various sections, may comprise the steps of:
i. providing a mold comprising a non-porous pattern, typically said pattern having the shape of the desired channel(s), and porous depressions outside of this pattern and in fluid communication with an under-pressure source;
ii. applying a first nonwoven web to said mold;
iii. applying an absorbent material over at least a portion of said nonwoven web, the under-pressure source being arranged to provide a vacuum force forcing said absorbent material into the porous depressions;
iv. applying a second nonwoven web directly or indirectly over the absorbent material, or folding said first nonwoven web, such to sandwich said absorbent material between an upper and lower layers of said nonwoven web(s);
v. optionally applying a bonding step to form a laminate comprising said first nonwoven, said second nonwoven and said absorbent material therebetween;
vi. optionally removing said laminate from the mold to form an absorbent core comprising channel(s) having the shape of the non-porous pattern.

More preferably, the channel(s) are formed substantially only by the vacuum force and no additional mechanical action such as embossing.

Adhesive may be applied to the respective substrates via one or more adhesive applicators that may be arranged to apply a spray and/or slot-coating of adhesive on respective substrates.

The molds described herein above may be contained within the circumference of a rotating drum apparatus, said drum apparatus typically comprising a plurality of said molds along its circumference. Said drum apparatus may be integrated within existing apparatuses for forming absorbent core laminates. An advantage of such a simple arrangement is that it allows for the formation of such novel absorbent cores in a simple and effective manner without considerable capital investment to substantially change major parts of existing core forming equipment.

The disclosure also relates to the use of an absorbent core described in the previous sections herein in an absorbent article described above, for improved liquid distribution compared to the same absorbent article comprising a core free of channels.

### TEST METHODS

### Absorption Time Under Pressure test (AtuP):

This test measures the time needed to absorb an amount of liquid measured under specific pressure conditions. It determines the speed at which the absorbent material absorbs an amount of artificial urine under certain pressure conditions.

### Material

1. 250 ml graduated cylinder.
2. Analytical balance accuracy 0,01g.
3. Chronometer.
4. Apparatus:
   - Transparent methacrylate column made up of a tube with an internal diameter of 21 mm ± 1 mm and measuring 650 mm ± 5mm in length placed in an inox holder with 107 mm ± 1mm diameter round base plate of 10 mm thickness. The methacrylate column is fixed such that 2 mm of the tube sticks through at the bottom of the iron base plate.
   - Cast iron weight placed over the methacrylate column.
   - The total weight of the apparatus is 3.7 kg ± 2%.
   - The total pressure to be applied onto the absorbent material is 0.585 psi.
5. Laboratory stand with locking forceps and arms.
6. 0.9 % saline solution.

### Procedure

1. Spread the absorbent material to be tested with the absorbent side facing upwards.
2. Mark the point at which the liquid enters the material; to do so, measure midway across the length & width in the crotch area of the absorbent core. In the examples 1 to 7 below, this point was set at the midway point across the length & width of the rectangular area in the crotch area showing the narrowest absorbent core width.
3. Place the methacrylate column with the weights on top of the absorbent core, matching the point marked on the absorbent material with the center of the column; stretch the absorbent material slightly to eliminate any possible wrinkles that might remain underneath or beside the column.
4. Pour 250 ml of saline solution into the tube of the methacrylate column and simultaneously start the chronometer.
5. Measure the time needed to absorb all the liquid.

### Rewet Under Pressure test (RuP):

This test measures the amount of liquid an absorbent material is incapable of retaining, as measured under specific pressure conditions. It determines the rewet of the absorbent material, by its ability to retain an amount of artificial urine without leaking through under certain time and pressure conditions.

### Material

1. 250 ml graduated cylinder.
2. Analytical balance accuracy 0,01g.
3. Electronic timers.
4. Apparatus: identical to the apparatus of the AtuP test above
5. Laboratory stand with locking forceps and arms.
6. Methacrylate disc measuring 90 mm in diameter by 10 mm thick; total weight 89 g ± 2%.
7. 10 kg ± 1% weight with diameter 120 mm ± 1 mm & 110 mm height (with a handling stand).
   The total pressure exerted on the filter paper is 2.2 psi.
8. Pneumatic device for automatic lowering & lifting the 10kg weight.
9. Whatmann N°1 110-mm round filter paper discs.
10. 0.9 % saline solution

### Procedure

1. Spread the absorbent material to be tested with the absorbent side facing upwards.
2. Mark the point at which the liquid enters the material; to do so, measure midway across the length & width in the crotch area of the absorbent core. In the examples 1 to 7 below, this point was set at the midway point across the length & width of the rectangular area in the crotch area showing the narrowest absorbent core width.
3. Place the methacrylate column with the weights on top of the absorbent core, matching the point marked on the absorbent material with the center of the column; stretch the absorbent material slightly to eliminate any possible wrinkles that might remain underneath or beside the column.
4. Pour 250 ml of saline solution into the tube of the methacrylate column and simultaneously start the timer.
5. During a 10 minutes period of time prepare ±25 g filter paper discs to measure dryness; weigh them, record this weight to the nearest 0.01g (Wd).
6. Once the 10 minutes have passed, remove the column. Immediately take the product and place it on the handling stand of the 10 kg weight. Avoid handling the absorbent material (product must stay flat).
7. Place the filter paper discs on the point marked; place the 90-mm round methacrylate disc on top and lower the weight automatically. Lowering the weight must happen smoothly and at low speed (max speed of 10mm / sec). Start the timer when the 10 kg weight reaches the methacrylate plate and the filterpapers are loaded. Keep the weight in place for 30 seconds.
8. Lift the weight and remove the disc; take the filter paper discs and weigh them; record this weight to the nearest 0.01 g (Ww).
9. RuP = Ww - Wd

### EXAMPLES

### Examples 1 to 5:

5 different absorbent cores according to the invention have been incorporated into 5 incontinence adult pants having the same topsheet, ADL and backsheet. These products have then been subjected to the Absorption Time Under Pressure test (AtuP) and the Rewet Under Pressure test (RuP) described above.

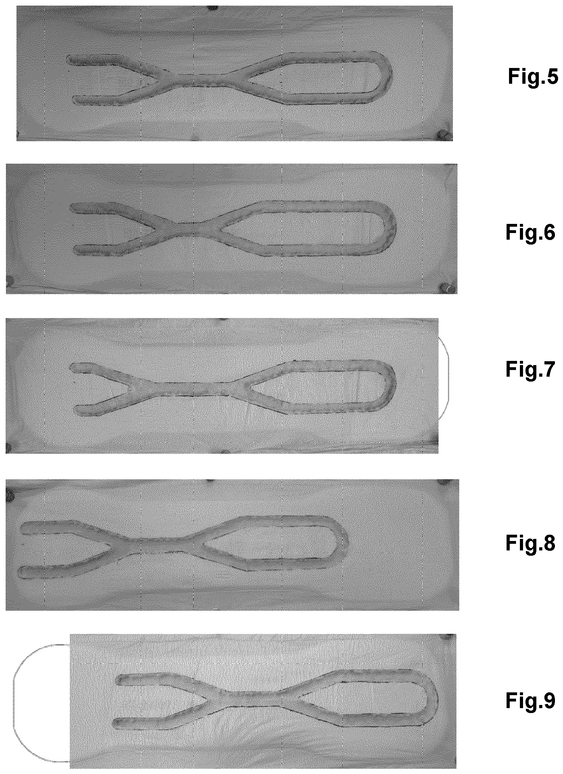

Examples 1 to 5 correspond respectively to Figures 5 to 9. In all the figures the back portion of the core is on the right. On figures 7 and 9, the core is not fully visible on the picture. The contour of the core has thus been complemented manually.

All the examples 1 to 5 have an absorbent core length of around 440 mm and a channel length of about 320 mm.

Example 1 is characterised by a central section of around 49 mm, Example 2 by a shorter central section of around 24.5 mm, and Example 3 by a longer central section of around 73.5 mm.

Example 1 is characterised by a central section longitudinally centered on a point which is at a distance from the front transverse edge of around 195 mm, i.e. of around 44% of the absorbent core length. For example 4, this point is closer to the front transverse edge, at around 155 mm; for example 5, this point is farther from the front transverse edge, at around 235 mm.

All the other features and dimensions of the channel are the same throughout examples 1 to 5.

The results of the tests are given in Table 1:

| **TABLE 1** | RuP [g] | AtuP [sec] |
|---|---|---|
| Example 1 - Fig.5 | 1.10 | 70.51 |
| Example 2 - Fig.6 | 1.26 | 99.52 |
| Example 3 - Fig.7 | 3.43 | 204.22 |
| Example 4 - Fig.8 | 1.17 | 99.76 |
| Example 5 - Fig.9 | 1.09 | 82.16 |

These results may be compared to deduct trends, but their absolute values should not be used to draw any conclusion, since other parts of the pant, for example the ADL, have not been optimised in these examples.

When comparing example 1 with examples 2 and 3, this shows that shortening the central section increases to some extent the rewet and the absorption time, and that lengthening the central section greatly increases both the rewet and the absorption time.

When comparing example 1 with examples 4 and 5, this shows that the positioning of the channel within the core, and in particular the positioning of the point on which the central section is centered, has an influence mainly on the absorption time.

### Examples 6 and 7:

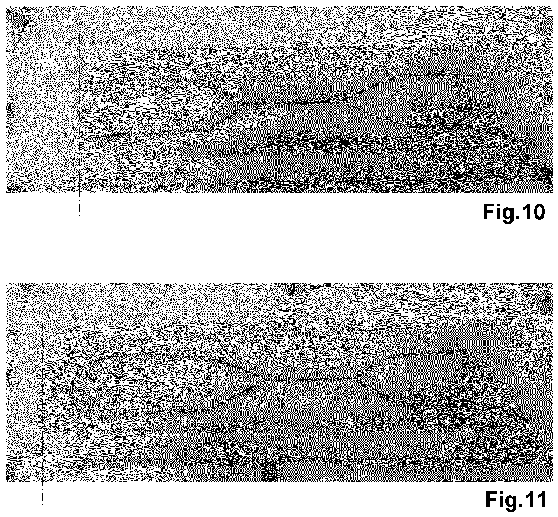

2 different absorbent cores have been incorporated into 2 incontinence adult pants having the same topsheet, ADL and backsheet, but different than in the examples 1 to 5 above. Examples 6 and 7 have an absorbent core length of around 440 mm and a channel length of about 320 mm. Example 6 corresponds to Figure 10 and is not a channel in accordance with the present invention. Example 7 corresponds to Figure 11 and is in accordance with the present invention. In these figures the back portion of the core is on the left and the channel shape has been highlighted with a marker pen. These products have been subjected to the Absorption Time Under Pressure test (AtuP) and the Rewet Under Pressure test (RuP) described above. Moreover the distribution of liquid has been observed and pictures of figures 10 and 11 have been taken after the Absorption Time Under Pressure test.

The results of the tests are given in Table 2:

| **TABLE 2** | RuP [g] | AtuP [sec] |
|---|---|---|
| Example 6 - Fig.10 | 0.09 | 27.22 |
| Example 7 - Fig.11 | 0.09 | 27.33 |

The Absorption Time Under Pressure and the Rewet Under Pressure results are equally very good for these two examples. But the distribution of liquid shows a very clear advantage of having a closing section at the end of the lateral sections at the back of the absorbent core. Without the closing section the liquid distribution stops at the end points of the two lateral sections. This is shown on the figure by a vertical dashed line. With the closing section the liquid distribution goes more to the back of the absorbent core and only stops beyond the end points of the two lateral sections. This is shown on the figure by a vertical dashed line. In the front portion of the core, the distribution in both examples go beyond the lateral sections end points. This may be explained because the miction point is more in the front of the core.

### Examples 8 and 9:

2 different absorbent cores according to the invention have been manufactured for later incorporation into unisex baby diapers. Examples 8 and 9 have an absorbent core length of around 360 mm and a channel length of about 325 mm.

Example 8, corresponding to Figure 3, is characterised by a central section closest to the transverse center line, of around 40 mm. Said central section is longitudinally centered on a point which is at a distance from the front transverse edge of around 190 mm.

Example 9, corresponding to Figure 4, is characterised by a central section crossing the transverse center line, of around 60 mm. Said central section is longitudinally centered on a point which is at a distance from the front transverse edge of around 190 mm.lt is supposed that the present disclosure is not restricted to any form of realization described previously and that some modifications can be added to the presented example of fabrication without reappraisal of the appended claims. For example, although the above examples make reference to a channel pattern of the type of Fig. 1, similar properties may be observed with other channel patterns as illustrated in Fig. 2 to Fig. 4, Moreover, although the example and associated figures relate to incontinence diapers, the same remains applicable to baby diapers and pants, or feminine hygiene products albeit with some structural alterations which would be apparent to a person skilled in the art.

## Claims

1. An absorbent article comprising a liquid permeable topsheet, a liquid impermeable backsheet and an absorbent core (1) positioned in between said topsheet and said backsheet, said absorbent core (1) having a first and second longitudinal edge (2,2') and a front and back transverse edge (3,3'), said absorbent core (1) having a longitudinal center line (LCL) dividing the absorbent core (1) in a first longitudinal portion and a second longitudinal portion on either side of the longitudinal center line (LCL), said absorbent core (1) having a transverse center line (TCL) dividing the absorbent core (1) in a front portion and a back portion on either side of the transverse center line (TCL), thereby forming four quadrants, wherein the absorbent core (1) comprises absorbent material selected from the group consisting of cellulose fibers, superabsorbent polymers and combinations thereof, wherein said absorbent material is contained within at least one core wrap substrate enclosing said absorbent material, and wherein a top layer of said core wrap is adhered to a bottom layer of said core wrap to form one or more channels (4) substantially free of said absorbent material, wherein at least one channel (4) follows a substantially continuous path from any point of said channel to any other point of the same channel, **characterized in that** said channel (4) consists of:
- one longitudinally extending central section (41) coincident with the longitudinal center line (LCL),
- one longitudinally extending lateral section (42) in each of the four quadrants,
- four diagonally extending diagonal sections (43), diverging from the central section end points (411,412) towards the lateral sections in each quadrant and connecting said central (41) and lateral (42) sections,
- one transversally extending closing section (44,44'), in one of the front or back portions, joining each of the two lateral sections end points (421,422) closest to the absorbent core respective front or back transverse edge (3,3'), and
- optionally a second transversally extending closing section (44,44'), in the other of the front or back portions, joining each of the two lateral sections end points (421,422) closest to the absorbent core respective front or back transverse edge (3,3'),
and **characterized in that** the central section (41) has a length (413) of between 4 and 25% of the absorbent core length (L).

2. An absorbent article comprising a liquid permeable topsheet, a liquid impermeable backsheet and an absorbent core (1) positioned in between said topsheet and said backsheet, said absorbent core (1) having a first and second longitudinal edge (2,2') and a front and back transverse edge (3,3'), said absorbent core (1) having a longitudinal center line (LCL) dividing the absorbent core (1) in a first longitudinal portion and a second longitudinal portion on either side of the longitudinal center line (LCL), said absorbent core (1) having a transverse center line (TCL) dividing the absorbent core (1) in a front portion and a back portion on either side of the transverse center line (TCL), thereby forming four quadrants, wherein the absorbent core (1) comprises absorbent material selected from the group consisting of cellulose fibers, superabsorbent polymers and combinations thereof, wherein said absorbent material is contained within at least one core wrap substrate enclosing said absorbent material, and wherein a top layer of said core wrap is adhered to a bottom layer of said core wrap to form one or more channels (4) substantially free of said absorbent material, wherein at least one channel (4) follows a substantially continuous path from any point of said channel to any other point of the same channel,
**characterized in that** said channel (4) comprises at least:
- one longitudinally extending central section (41) coincident with the longitudinal center line (LCL),
- one longitudinally extending lateral section (42) in each of the four quadrants,
- four diagonally extending diagonal sections (43), diverging from the central section end points (411,412) towards the lateral sections in each quadrant and connecting said central (41) and lateral (42) sections,
- one transversally extending closing section (44,44'), in one of the front or back portions, joining each of the two lateral sections end points (421,422) closest to the absorbent core respective front or back transverse edge (3,3'), and
- a second transversally extending closing section (44,44'), in the other of the front or back portions, joining each of the two lateral sections end points (421,422) closest to the absorbent core respective front or back transverse edge (3,3'),
wherein said channel (4) comprises at least 2 central sections (41), 6 lateral sections (42), 8 diagonal sections (43) and two closing sections (44),
and **characterized in that** the central section (41) closest to or crossing the transverse center line (TCL) has a length (413) of between 5 and 25% of the absorbent core length (L).

3. An absorbent article according to claim 1, wherein the closing section (44) is in the back portion of the absorbent core (1).

4. An absorbent article according to claim 1 or claim 3, wherein the central section (41) is longitudinally centered on a point (414) which is at a distance from the front transverse edge (3) along the longitudinal center line (LCL) of between 30% and 55% of the absorbent core length (L).

5. An absorbent article according to claim 1 or claim 3 or claim 4, wherein the length of the channel (CL) taken along the longitudinal center line (LCL) is between 50 and 90% of the absorbent core length (L).

6. An absorbent article according to claim 2, wherein the central section (41) closest to or crossing the transverse center line (TCL) is longitudinally centered on a point (414) which is at a distance from the front transverse edge (3) along the longitudinal center line (LCL) of between 40% and 65% of the absorbent core length (L).

7. An absorbent article according to claim 2 or claim 6, wherein said channel (4) comprises at least two diverging sections (45) connecting lateral sections (42) and/or diagonal sections (43) to the longitudinal edges (2,2') of the absorbent core (1).

8. An absorbent article according to claim 2 or claim 6 or claim 7, wherein the length of the channel (CL) taken along the longitudinal center line (LCL) is between 70 and 95% of the absorbent core length (L).

9. An absorbent article according to any of the preceding claims, wherein the absorbent core (1) is free of any channel or channel section extending up to the front and back transverse edges (3,3') of the absorbent core (1).

10. An absorbent article according to any of the preceding claims, wherein the lateral sections (42) of the first longitudinal portion are aligned on a first longitudinal axis parallel to the longitudinal center line (LCL), and the lateral sections (42) of the second longitudinal portion are aligned on a second longitudinal axis parallel to the longitudinal center line (LCL).

11. An absorbent article according to any of the preceding claims, wherein an acquisition distribution system is present between the topsheet and the absorbent core, said system being a single layer of spunbond and/or carded nonwoven.

12. An absorbent article according to any of the claims 1 to 10, wherein an acquisition distribution system is present between the topsheet and the absorbent core, said system being multi-layered and comprising at least one spunbond layer and at least one meltblown layer.

13. An absorbent article according to any of the claims 1 to 10, wherein the top layer of the core wrap is in direct contact with the topsheet and comprises a spunbond and/or carded nonwoven layer.

14. An absorbent article according to any of the claims 1 to 10, wherein the top layer of the core wrap is in direct contact with the topsheet, is multi-layered and comprises at least one spunbond layer and at least one meltblown layer.

## Patentansprüche

1. Absorbierender Artikel, umfassend eine flüssigkeitsdurchlässige Oberschicht, eine flüssigkeitsundurchlässige Rückschicht und einen absorbierenden Kern (1), der zwischen der Oberschicht und der Rückschicht positioniert ist, wobei der absorbierende Kern (1) eine erste und eine zweite Längskante (2,2') und eine vorderseitige und eine rückseitige Querkante (3,3') aufweist, wobei der absorbierende Kern (1) eine Längsmittellinie (LCL) aufweist, die den absorbierenden Kern (1) in einen ersten Längsabschnitt und einen zweiten Längsabschnitt auf beiden Seiten der Längsmittellinie (LCL) unterteilt, wobei der absorbierende Kern (1) eine Quermittellinie (TCL) aufweist, die den absorbierenden Kern (1) in einen vorderseitigen Abschnitt und einen rückseitigen Abschnitt auf beiden Seiten der Quermittellinie (TCL) unterteilt, wobei dadurch vier Quadranten ausgebildet werden, wobei der absorbierende Kern (1) absorbierendes Material umfasst, das aus der Gruppe ausgewählt ist, bestehend aus Zellulosefasern, superabsorbierenden Polymeren und Kombinationen davon, wobei das absorbierende Material innerhalb mindestens eines Kernumhüllungssubstrats enthalten ist, das das absorbierende Material umschließt, und wobei eine obere Lage der Kernumhüllung an eine untere Lage der Kernumhüllung geklebt ist, um einen oder mehrere Kanäle (4) auszubilden, die im Wesentlichen frei von dem absorbierenden Material sind, wobei mindestens ein Kanal (4) einem im Wesentlichen kontinuierlichen Weg von einem beliebigen Punkt des Kanals zu einem beliebigen anderen Punkt desselben Kanals folgt, **dadurch gekennzeichnet, dass** der Kanal (4) besteht aus:
- einem sich in Längsrichtung erstreckenden mittleren Teil (41), der mit der Längsmittellinie (LCL) zusammenfällt,
- einem sich in Längsrichtung erstreckenden lateralen Teil (42) in jedem der vier Quadranten,
- vier sich diagonal erstreckenden diagonalen Teilen (43), die von den Endpunkten (411,412) des mittleren Teils zu den lateralen Teilen in jedem Quadranten hin divergieren und die mittleren (41) und lateralen (42) Teile verbinden,
- einem sich quer erstreckenden Abschlussteil (44,44'), in einem des vorderseitigen oder des rückseitigen Abschnitts, der jeden der zwei Endpunkte (421,422) der lateralen Teile, die der jeweiligen vorderseitigen oder rückseitigen Querkante (3,3') des absorbierenden Kerns am nächsten sind, verbindet, und
- optional einem zweiten sich quer erstreckenden Abschlussteil (44,44') in dem anderen des vorderseitigen oder des rückseitigen Abschnitts, der jeden der zwei Endpunkte (421,422) der lateralen Teile, die der jeweiligen vorderseitigen oder rückseitigen Querkante (3,3') des absorbierenden Kerns am nächsten sind, verbindet,
und **dadurch gekennzeichnet, dass** der mittlere Teil (41) eine Länge (413) zwischen 4 und 25 % der Länge (L) des absorbierenden Kerns aufweist.

2. Absorbierender Artikel, umfassend eine flüssigkeitsdurchlässige Oberschicht, eine flüssigkeitsundurchlässige Rückschicht und einen absorbierenden Kern (1), der zwischen der Oberschicht und der Rückschicht positioniert ist, wobei der absorbierende Kern (1) eine erste und eine zweite Längskante (2,2') und eine vorderseitige und eine rückseitige Querkante (3,3') aufweist, wobei der absorbierende Kern (1) eine Längsmittellinie (LCL) aufweist, die den absorbierenden Kern (1) in einen ersten Längsabschnitt und einen zweiten Längsabschnitt auf beiden Seiten der Längsmittellinie (LCL) unterteilt, wobei der absorbierende Kern (1) eine Quermittellinie (TCL) aufweist, die den absorbierenden Kern (1) in einen vorderseitigen Abschnitt und einen rückseitigen Abschnitt auf beiden Seiten der Quermittellinie (TCL) unterteilt, wobei dadurch vier Quadranten ausgebildet werden, wobei der absorbierende Kern (1) absorbierendes Material umfasst, das aus der Gruppe ausgewählt ist, bestehend aus Zellulosefasern, superabsorbierenden Polymeren und Kombinationen davon, wobei das absorbierende Material innerhalb mindestens eines Kernumhüllungssubstrats enthalten ist, das das absorbierende Material umschließt, und wobei eine obere Lage der Kernumhüllung an eine untere Lage der Kernumhüllung geklebt ist, um einen oder mehrere Kanäle (4) auszubilden, die im Wesentlichen frei von dem absorbierenden Material sind, wobei mindestens ein Kanal (4) einem im Wesentlichen kontinuierlichen Weg von einem beliebigen Punkt des Kanals zu einem beliebigen anderen Punkt desselben Kanals folgt, **dadurch gekennzeichnet, dass** der Kanal (4) mindestens umfasst:
- einen sich in Längsrichtung erstreckenden mittleren Teil (41), der mit der Längsmittellinie (LCL) zusammenfällt,
- einen sich in Längsrichtung erstreckenden lateralen Teil (42) in jedem der vier Quadranten,
- vier sich diagonal erstreckende diagonale Teile (43), die von den Endpunkten (411,412) des mittleren Teils zu den lateralen Teilen in jedem Quadranten hin divergieren und die mittleren (41) und lateralen (42) Teile verbinden,
- einen sich quer erstreckenden Abschlussteil (44,44'), in einem des vorderseitigen oder des rückseitigen Abschnitts, der jeden der zwei Endpunkte (421,422) der lateralen Teile, die der jeweiligen vorderseitigen oder rückseitigen Querkante (3,3') des absorbierenden Kerns am nächsten sind, verbindet, und
- einen zweiten sich quer erstreckenden Abschlussteil (44,44') in dem anderen des vorderseitigen oder des rückseitigen Abschnitts, der jeden der zwei Endpunkte (421,422) der lateralen Teile, die der jeweiligen vorderseitigen oder rückseitigen Querkante (3,3') des absorbierenden Kerns am nächsten sind, verbindet,
wobei der Kanal (4) mindestens 2 mittlere Teile (41), 6 laterale Teile (42), 8 diagonale Teile (43) und zwei Abschlussteile (44) umfasst,
und **dadurch gekennzeichnet, dass** der mittlere Teil (41), der der Quermittellinie (TCL) am nächsten ist oder diese kreuzt, eine Länge (413) zwischen 5 und 25 % der Länge (L) des absorbierenden Kerns aufweist.

3. Absorbierender Artikel nach Anspruch 1, wobei sich der Abschlussteil (44) in dem rückseitigen Abschnitt des absorbierenden Kerns (1) befindet.

4. Absorbierender Artikel nach Anspruch 1 oder 3, wobei der mittlere Teil (41) auf einen Punkt (414) in Längsrichtung mittig eingestellt ist, der sich in einem Abstand von der vorderseitigen Querkante (3) entlang der Längsmittellinie (LCL) von zwischen 30 % und 55 % der Länge (L) des absorbierenden Kerns befindet.

5. Absorbierender Artikel nach Anspruch 1 oder 3 oder 4, wobei die Länge des Kanals (CL), entlang der Längsmittellinie (LCL) genommen, zwischen 50 und 90 % der Länge (L) des absorbierenden Kerns beträgt.

6. Absorbierender Artikel nach Anspruch 2, wobei der mittlere Teil (41), der der Quermittellinie (TCL) am nächsten ist oder diese kreuzt, auf einen Punkt (414) in Längsrichtung mittig eingestellt ist, der sich in einem Abstand von der vorderseitigen Querkante (3) entlang der Längsmittellinie (LCL) von zwischen 40 % und 65 % der Länge (L) des absorbierenden Kerns befindet.

7. Absorbierender Artikel nach Anspruch 2 oder 6, wobei der Kanal (4) mindestens zwei divergierende Teile (45) umfasst, die die lateralen Teile (42) und/oder diagonalen Teile (43) mit den Längsrändern (2,2') des absorbierenden Kerns (1) verbinden.

8. Absorbierender Artikel nach Anspruch 2 oder 6 oder 7, wobei die Länge des Kanals (CL), entlang der Längsmittellinie (LCL) genommen, zwischen 70 und 95 % der Länge (L) des absorbierenden Kerns beträgt.

9. Absorbierender Artikel nach einem der vorstehenden Ansprüche, wobei der absorbierende Kern (1) frei von einem beliebigen Kanal oder Kanalteil ist, der sich bis zu der vorderseitigen oder der rückseitigen Querkante (3,3') des absorbierenden Kerns (1) erstreckt.

10. Absorbierender Artikel nach einem der vorstehenden Ansprüche, wobei die lateralen Teile (42) des ersten Längsabschnitts auf einer ersten Längsachse parallel zu der Längsmittellinie (LCL) ausgerichtet sind und die lateralen Teile (42) des zweiten Längsabschnitts auf einer zweiten Längsachse parallel zu der Längsmittellinie (LCL) ausgerichtet sind.

11. Absorbierender Artikel nach einem der vorstehenden Ansprüche, wobei ein Aufnahme-Verteilungssystem zwischen der Oberschicht und dem absorbierenden Kern vorhanden ist, wobei das System eine einzelne Lage aus Spinnvlies und/oder kardiertem Vlies ist.

12. Absorbierender Artikel nach einem der Ansprüche 1 bis 10, wobei ein Aufnahme-Verteilungssystem zwischen der Oberschicht und dem absorbierenden Kern vorhanden ist, wobei das System mehrlagig ist und mindestens eine Spinnvlieslage und mindestens eine schmelzgeblasene Lage umfasst.

13. Absorbierender Artikel nach einem der Ansprüche 1 bis 10, wobei die obere Lage der Kernumhüllung in direktem Kontakt mit der Oberschicht steht und eine Spinnvlies- und/oder kardierte Vlieslage umfasst.

14. Absorbierender Artikel nach einem der Ansprüche 1 bis 10, wobei die obere Lage der Kernumhüllung in direktem Kontakt mit der Oberschicht steht, mehrlagig ist und mindestens eine Spinnvlieslage und mindestens eine schmelzgeblasene Lage umfasst.

## Revendications

1. Article absorbant comprenant une feuille supérieure perméable aux liquides, une feuille arrière imperméable aux liquides et une âme absorbante (1) placée entre ladite feuille supérieure et ladite feuille arrière, ladite âme absorbante (1) ayant un premier et un second bord longitudinal (2, 2') et un bord transversal avant et un bord transversal arrière (3, 3'), ladite âme absorbante (1) ayant une ligne centrale longitudinale (LCL) divisant l'âme absorbante (1) en une première partie longitudinale et une seconde partie longitudinale de part et d'autre de la ligne centrale longitudinale (LCL), ladite âme absorbante (1) ayant une ligne centrale transversale (TCL) divisant l'âme absorbante (1) en une partie avant et une partie arrière de part et d'autre de la ligne centrale transversale (TCL), formant ainsi quatre quadrants, dans lequel l'âme absorbante (1) comprend un matériau absorbant choisi dans le groupe constitué de fibres de cellulose, de polymères superabsorbants et de combinaisons de ceux-ci, dans lequel ledit matériau absorbant est contenu dans au moins un substrat d'enveloppe d'âme renfermant ledit matériau absorbant, et dans lequel une couche supérieure de ladite enveloppe d'âme est collée à une couche inférieure de ladite enveloppe d'âme pour former un ou plusieurs canaux (4) sensiblement exempts dudit matériau absorbant, dans lequel au moins un canal (4) suit un chemin sensiblement continu depuis n'importe quel point dudit canal jusqu'à n'importe quel autre point du même canal, **caractérisé en ce que** ledit canal (4) est constitué de :
- une section centrale (41) s'étendant longitudinalement et coïncidant avec la ligne centrale longitudinale (LCL),
- une section latérale (42) s'étendant longitudinalement dans chacun des quatre quadrants,
- quatre sections diagonales (43) s'étendant en diagonal, s'écartant des points d'extrémité de la section centrale (411, 412) en direction des sections latérales de chaque quadrant et reliant lesdites sections centrales (41) et latérales (42),
- une section de fermeture (44, 44') s'étendant transversalement, dans l'une des parties avant ou arrière, reliant chacun des deux points d'extrémité des sections latérales (421, 422) les plus proches du bord transversal avant ou arrière respectif (3, 3') de l'âme absorbante, et
- éventuellement, une deuxième section de fermeture (44, 44') s'étendant transversalement, dans l'autre des parties avant ou arrière, reliant chacun des deux points d'extrémité des sections latérales (421, 422) les plus proches du bord transversal avant ou arrière respectif (3, 3') de l'âme absorbante,
et **caractérisé en ce que** la section centrale (41) a une longueur (413) comprise entre 4 et 25 % de la longueur (L) de l'âme absorbante.

2. Article absorbant comprenant une feuille supérieure perméable aux liquides, une feuille arrière imperméable aux liquides et une âme absorbante (1) placée entre ladite feuille supérieure et ladite feuille arrière, ladite âme absorbante (1) ayant un premier et un second bord longitudinal (2, 2') et un bord transversal avant et un bord transversal arrière (3, 3'), ladite âme absorbante (1) ayant une ligne centrale longitudinale (LCL) divisant l'âme absorbante (1) en une première partie longitudinale et une seconde partie longitudinale de part et d'autre de la ligne centrale longitudinale (LCL), ladite âme absorbante (1) ayant une ligne centrale transversale (TCL) divisant l'âme absorbante (1) en une partie avant et une partie arrière de part et d'autre de la ligne centrale transversale (TCL), formant ainsi quatre quadrants, dans lequel l'âme absorbante (1) comprend un matériau absorbant choisi dans le groupe constitué de fibres de cellulose, de polymères superabsorbants et de combinaisons de ceux-ci, dans lequel ledit matériau absorbant est contenu dans au moins un substrat d'enveloppe d'âme renfermant ledit matériau absorbant, et dans lequel une couche supérieure de ladite enveloppe d'âme est collée à une couche inférieure de ladite enveloppe d'âme pour former un ou plusieurs canaux (4) sensiblement exempts dudit matériau absorbant, dans lequel au moins un canal (4) suit un chemin sensiblement continu depuis n'importe quel point dudit canal jusqu'à n'importe quel autre point du même canal, **caractérisé en ce que** ledit canal (4) comprend au moins :
- une section centrale (41) s'étendant longitudinalement et coïncidant avec la ligne centrale longitudinale (LCL),
- une section latérale (42) s'étendant longitudinalement dans chacun des quatre quadrants,
- quatre sections diagonales (43) s'étendant en diagonal, s'écartant des points d'extrémité de la section centrale (411, 412) en direction des sections latérales de chaque quadrant et reliant lesdites sections centrales (41) et latérales (42),
- une section de fermeture (44, 44') s'étendant transversalement, dans l'une des parties avant ou arrière, reliant chacun des deux points d'extrémité des sections latérales (421, 422) les plus proches du bord transversal avant ou arrière respectif (3, 3') de l'âme absorbante, et
- une deuxième section de fermeture (44, 44') s'étendant transversalement, dans l'autre des parties avant ou arrière, reliant chacun des deux points d'extrémité des sections latérales (421, 422) les plus proches du bord transversal avant ou arrière respectif (3, 3') de l'âme absorbante,
dans lequel ledit canal (4) comprend au moins 2 sections centrales (41), 6 sections latérales (42), 8 sections diagonales (43) et deux sections de fermeture (44),
et **caractérisée en ce que** la section centrale (41) la plus proche de la ligne centrale transversale (TCL) ou traversant celle-ci a une longueur (413) comprise entre 5 et 25 % de la longueur (L) de l'âme absorbante.

3. Article absorbant selon la revendication 1, dans lequel la section de fermeture (44) se trouve dans la partie arrière de l'âme absorbante (1).

4. Article absorbant selon la revendication 1 ou la revendication 3, dans lequel la section centrale (41) est centrée longitudinalement sur un point (414) qui se trouve à une distance du bord transversal avant (3) le long de la ligne centrale longitudinale (LCL) comprise entre 30 % et 55 % de la longueur de l'âme absorbante (L).

5. Article absorbant selon la revendication 1, la revendication 3 ou la revendication 4, dans lequel la longueur du canal (CL) mesurée le long de la ligne centrale longitudinale (LCL) est comprise entre 50 et 90 % de la longueur de l'âme absorbante (L).

6. Article absorbant selon la revendication 2, dans lequel la section centrale (41) la plus proche de la ligne centrale transversale (TCL) ou traversant celle-ci est longitudinalement centrée sur un point (414) qui est à une distance du bord transversal avant (3) le long de la ligne centrale longitudinale (LCL) comprise entre 40 % et 65 % de la longueur de l'âme absorbante (L).

7. Article absorbant selon la revendication 2 ou la revendication 6, dans lequel ledit canal (4) comprend au moins deux sections divergentes (45) reliant des sections latérales (42) et/ou diagonales (43) aux bords longitudinaux (2, 2') de l'âme absorbante (1).

8. Article absorbant selon la revendication 2, la revendication 6 ou la revendication 7, dans lequel la longueur du canal (CL) le long de la ligne centrale longitudinale (LCL) est comprise entre 70 et 95 % de la longueur de l'âme absorbante (L).

9. Article absorbant selon l'une quelconque des revendications précédentes, dans lequel l'âme absorbante (1) est exempte de tout canal ou section de canal s'étendant jusqu'aux bords transversaux avant et arrière (3, 3') de l'âme absorbante (1).

10. Article absorbant selon l'une quelconque des revendications précédentes, dans lequel les sections latérales (42) de la première partie longitudinale sont alignées sur un premier axe longitudinal parallèle à la ligne centrale longitudinale (LCL), et les sections latérales (42) de la deuxième partie longitudinale sont alignées sur un deuxième axe longitudinal parallèle à la ligne centrale longitudinale (LCL).

11. Article absorbant selon l'une quelconque des revendications précédentes, dans lequel un système de récupération répartition est présent entre la feuille supérieure et l'âme absorbante, ledit système étant une monocouche de non-tissé filé-lié et/ou cardé.

12. Article absorbant selon l'une quelconque des revendications 1 à 10, dans lequel un système de récupération répartition est présent entre la feuille supérieure et l'âme absorbante, ledit système étant multicouche et comprenant au moins une couche filée-liée et au moins une couche extrudée-soufflée.

13. Article absorbant selon l'une quelconque des revendications 1 à 10, dans lequel la couche supérieure de l'enveloppe d'âme est en contact direct avec la feuille supérieure et comprend une couche de non-tissé filé-lié et/ou cardé.

14. Article absorbant selon l'une quelconque des revendications 1 à 10, dans lequel la couche supérieure de l'enveloppe d'âme est en contact direct avec la feuille supérieure, est multicouche et comprend au moins une couche filée-liée et au moins une couche extrudée-soufflée.
